# EUROPEAN PATENT APPLICATION

(11) **EP 3 109 639 A1**
(43) Date of publication of application: **28.12.2016**
(21) Application number: 15174020.6
(22) Date of filing: 26.06.2015
(51) Int. Cl.: G01N 33/68

(54) **MASS SPECTROMETRY QUANTIFICATION**

(71) Applicant: Atlas Antibodies AB, 106 91 Stockholm (SE)
(72) Inventor: Forsström, Björn, 16 66 Stockholm (SE); Edfors, Fredrik, 115 38 Stockholm (SE); Uhlén, Mathias, 114 25 Stockholm (SE)
(74) Representative: Kransell & Wennborg KB

(57) **Abstract**

There is provided a method of determining the absolute amount of at least one target protein in a sample, said method comprising the following steps:
(a) adding an absolute amount of a mass-altered standard protein for each target protein to said sample;
(b) proteolytically cleaving target protein and standard protein from step (a) using at least one protease to obtain cleaved peptides;
(c) subjecting cleaved peptides from step (b), optionally after chromatography, to mass spectrometry (MS) to obtain a spectrum, which MS comprises filtering using an isolation window of 1-3 Th for co-isolation of ions from each pair of target protein and standard protein; and
(d) determining the absolute amount of said target protein(s) in said sample from the spectrum obtained in step (c) and said absolute amount(s) of corresponding standard protein(s) added in step (a), wherein
(i) each standard protein comprises a target sequence that is identical to a subsequence of its target protein,
(ii) the target sequence comprises at least one cleavage amino acid residue at which the at least one protease cleaves,
(iii) the mass of each cleavage amino acid residue in the target sequence of the standard protein is altered by 1-5 Da, such as 1-4 Da or 1-2 Da, with respect to the mass of the corresponding cleavage amino acid residue(s) in the subsequence of the target protein and
(iv) the amino acid residues in the target sequence of the standard protein other than the cleavage amino acid residue(s) are not mass altered with respect to the corresponding amino acid residues in the subsequence of the target protein. An isotopically labelled fusion protein that can be used in the method is also provided.

## Description

### Technical field

The present invention relates to the field of quantification of proteins of interest, e.g. in a crude sample. The quantification is based on the use of mass-altered standards in combination with mass spectrometry (MS).

### Background

Stable isotope-labeled (SIL) standards are used as internal standards in mass spectrometry, which allows for absolute protein quantification. One example is SIL standards suitable for use with trypsin digested samples. Such SIL standards normally comprise labeled lysines and arginines, containing ¹³C and ¹⁵N atoms, which introduce mass shifts of 8 and 10 Da, respectively. This difference is large enough for most mass spectrometers to distinguish between heavy (mass-altered) and light (not mass-altered) versions of the peptides and are suitable for e.g. for Selected Reaction Monitoring (SRM) where heavy and light peptide fragments are measured separately.

### Summary

The present inventors have realized that in Parallel Reaction Monitoring (PRM) using high-resolution mass spectrometers, fragments of heavy and light peptides can be analyzed simultaneously, which can allow for a more exact determination of the heavy-to-light ratio than when the fragments are measured at different times.

In order to be able to measure heavy and light peptide fragments simultaneously, the mass-to-charge (m/z) window in the first quadrupole mass filter has to be big enough to allow both heavy and light peptides to pass into the collision chamber for fragmentation and subsequent analysis by the high-resolution mass analyzer. However, when using SIL standards with mass shifts of 8 and 10 Da, this isolation window has to be set to more than 5 Th (rather 6 Th) for simultaneous isolation of heavy and light 2+ charged peptides. Such a large mass window can allow many other peptides to be co-isolated with those derived from the target proteins if they are eluted at the same time from a preceding liquid chromatography (LC) and fall within this isolation window. If the co-isolated peptides give rise to peptide fragments of similar m/z as the fragments of the target proteins, these fragments will interfere and give erroneous quantification of the target fragments.

The inventors have realized that a solution to this problem is to use standards with a very small mass shift compared to the endogenous light peptides. For example, lysine and arginine residues with heavy ¹⁵N, but normal ¹²C instead of heavy ¹³C, can be used in the standards. In such case, the mass shift per lysine and arginine containing peptide is 2 and 4 Da, respectively. This would in turn allow simultaneous isolation of heavy and light 2+ charged peptides using a window of only 2.5-3 m/z. This is just marginally larger than a window of 1-2 m/z normally used for isolation of heavy and light in separate windows and would substantially reduce the interfering signals compared to simultaneous isolation using a window of 6 m/z.

The inventors have further realized that one of the limiting parameters for multiplexing PRM assays is the number of overlapping scans the instrument has to perform simultaneously on peptides with similar LC retention times. The endogenous peptide and its corresponding SIL peptide co-elute and to study them with separate isolation windows means that the mass spectrometer would have to make twice the number of scans compared to if they were isolated in one window. Changing to simultaneous isolation would therefore double the multiplexing capacity.

An object of the present disclosure is thus to improve the precision of MS-based quantifications. Another object of the present disclosure is to decrease the time needed for MS quantifications.

The following is a non-limiting and itemized listing of embodiments of the present disclosure.
1. A method of determining the absolute amount of at least one target protein in a sample, said method comprising the following steps:
   (a) adding an absolute amount of a mass-altered standard protein for each target protein to said sample;
   (b) proteolytically cleaving target protein and standard protein from step (a) using at least one protease to obtain cleaved peptides;
   (c) subjecting cleaved peptides from step (b), optionally after chromatography, to mass spectrometry (MS) to obtain a spectrum, which MS comprises filtering using an isolation window of 1-3 Th for co-isolation of ions from each pair of target protein and standard protein; and
   (d) determining the absolute amount of said target protein(s) in said sample from the spectrum obtained in step (c) and said absolute amount(s) of corresponding standard protein(s) added in step (a), wherein
      (i) each standard protein comprises a target sequence that is identical to a subsequence of its target protein,
      (ii) the target sequence comprises at least one cleavage amino acid residue at which the at least one protease cleaves,
      (iii) the mass of each cleavage amino acid residue in the target sequence of the standard protein is altered by 1-5 Da, such as 1-4 Da or 1-2 Da, with respect to the mass of the corresponding cleavage amino acid residue(s) in the subsequence of the target protein and
      (iv) the amino acid residues in the target sequence of the standard protein other than the cleavage amino acid residue(s) are not mass altered with respect to the corresponding amino acid residues in the subsequence of the target protein.
2. The method according to item 1, wherein the absolute amounts of at least two, such as at least 5, such as at least 10 target proteins are determined.
3. The method according to any one of the preceding items, wherein the sample is an optionally diluted crude sample, such as an optionally diluted sample of cell culture supernatant, lysate, serum or plasma.
4. The method according to item 3, wherein the sample is albumin-depleted or non-depleted.
5. The method according to any one of the preceding items, wherein the at least one protease is trypsin and the at least one cleavage amino acid residue is selected from lysine and arginine.
6. The method according to item 5, wherein the at least one cleavage amino acid residue is lysine and arginine.
7. The method according to any one of the preceding items, wherein the mass of at least one N atom of the at least one cleavage amino acid residue is 15 Da in the target sequence of the standard protein and 14 Da in the subsequence of the target protein.
8. The method according to any one of the preceding items, wherein the only isotope labels of the target sequence of each standard protein are one or more ¹⁵N in each lysine and arginine, such as one or two ¹⁵N in each lysine and arginine.
9. The method according to any one of the preceding items, wherein each target sequence consists of 15-205 amino acid residues, such as 20-120 amino acid residues, such as 30-170 amino acid residues, such as 50-150 amino acid residues.
10. The method according to any one of the preceding items, wherein each target sequence comprises at least 2 cleavage amino acid residues, such as at least 3, 4, 5 or 6 cleavage amino acid residues.
11. The method according to any one of the preceding items, wherein each standard protein comprises 35-455 amino acid residues, such as 40-300 amino acid residues, such as 100-200 amino acid residues.
12. The method according to any one of the preceding items, wherein the standard protein is a fusion protein.
13. The method according to item 12, wherein the fusion protein comprises a solubility tag or a fragment thereof, wherein the solubility tag is selected from Maltose-binding protein (MBP), Glutathione-S-transferase (GST), Thioredoxin (Trx), N-Utilization substance (NusA), Small ubiquitin-modifier (SUMO), a Solubility-enhancing tag (SET), a Disulfide forming protein C (DsbC), Seventeen kilodalton protein (Skp), Phage T7 protein kinase (T7PK), Protein G B1 domain (GB1), Protein A IgG ZZ repeat domain (ZZ) and Albumin Binding Protein (ABP).
14. The method according to item 13, wherein the solubility tag or the fragment thereof comprises at least 15 amino acids.
15. The method according to any one of items 12-14, wherein the fusion protein comprises a purification tag selected from a His tag, a FLAG tag, a SBP tag, a myc tag and a OneStrep tag.
16. The method according to any one of the preceding items, wherein the mass spectrometry of step c) is tandem mass spectrometry (MS/MS), such as parallel reaction monitoring (PRM), involving filtering of precursor ions using an isolation window of 1-3 Th to obtain a spectrum of fragment ions.
17. An isotopically labelled fusion protein for the quantification of a target protein by mass spectrometry, wherein:
   said fusion polypeptide consists of 30-455 amino acid residues and comprises (i) a target sequence, which is identical to the target protein or a subsequence thereof, and (ii) a second sequence, which is not identical to the target protein or a subsequence thereof;
   said target sequence consists of 15-205 amino acid residues and comprises at least one amino acid residue mass-altered by 1-2 Da, which mass-altered amino acid residue(s) is/are selected from lysine and arginine in which one or two of the nitrogen atoms is/are ¹⁵N, wherein the amino acid residues in the target sequence other than lysine and arginine amino acid residues are not mass-altered; and
   said second sequence consists of 15-250 amino acid residues, such as 20-250 amino acid residues, such as 25-250 amino acid residues.
18. The isotopically labelled fusion protein of item 17, wherein said target sequence comprises at least 2, such as at least 3, 4, 5 or 6 amino acid residues mass-altered by 1-2 Da, which mass-altered amino acid residues are selected from lysine and arginine in which one or two of the nitrogen atoms is/are ¹⁵N.
19. The isotopically labelled fusion protein of item 17 or 18, wherein said target sequence comprises at least one arginine residue in which one or two of the nitrogen atoms is/are ¹⁵N.
20. The isotopically labelled fusion protein of any one of items 17-19, wherein said target sequence comprises at least two, such as at least three or four arginine residues in which one or two of the nitrogen atoms is/are ¹⁵N.
21. The isotopically labelled fusion protein of any one of items 17-20, consisting of 40-300 amino acid residues, such as 100-200 amino acid residues.
22. The isotopically labelled fusion protein of any one of items 17-21, wherein the target sequence consists of 20-200 amino acid residues, such as 30-170 amino acid residues, such as 50-150 amino acid residues.
23. The isotopically labelled fusion protein of any one of items 17-22, wherein the second sequence is a solubility tag or a fragment thereof and the solubility tag is selected from Maltose-binding protein (MBP), Glutathione-S-transferase (GST), Thioredoxin (Trx), N-Utilization substance (NusA), Small ubiquitin-modifier (SUMO), a Solubility-enhancing tag (SET), a Disulfide forming protein C (DsbC), Seventeen kilodalton protein (Skp), Phage T7 protein kinase (T7PK), Protein G B1 domain (GB1), Protein A IgG ZZ repeat domain (ZZ) and Albumin Binding Protein (ABP).
24. The isotopically labelled fusion protein of any one of items 17-22, further comprising a purification tag, such as a tag selected from a His tag, a FLAG tag, a SBP tag, a myc tag and a OneStrep tag.
25. A method of determining the absolute amount of at least one target protein in a sample, said method comprising the following steps:
   (a) proteolytically cleaving target protein from or in the sample using at least one protease to obtain cleaved peptides, wherein an absolute amount of a mass-altered standard peptide for each target protein is added before or after the proteolytic cleaving such that a mixture of cleaved peptides and standard peptide(s) is obtained after the proteolytic cleaving;
   (b) subjecting the mixture, optionally after chromatography, to mass spectrometry (MS) to obtain a spectrum, which MS comprises filtering using an isolation window of 1-3 Th for co-isolation of ions from each pair of target protein and standard peptide; and
   (c) determining the absolute amount of said target protein(s) in said sample from the spectrum obtained in step (b) and said absolute amount(s) of corresponding standard peptide(s) added in step (a), wherein
      (i) the amino acid sequence of each standard peptide is identical to a subsequence of its target protein and comprises at one of the terminal ends a cleavage amino acid residue at which the at least one protease cleaves,
      (ii) the mass of the cleavage amino acid residue in the standard peptide is altered by 1-5 Da, such as 1-4 Da or 1-2 Da, with respect to the mass of the corresponding cleavage amino acid residue(s) in the subsequence of the target protein and
      (iii) the amino acid residues in the standard peptide other than the cleavage amino acid residue are not mass altered with respect to the corresponding amino acid residues in the subsequence of the target protein.
26. The method according to item 25, wherein the absolute amounts of at least two, such as at least 5, such as at least 10 target proteins are determined.
27. The method according to item 25 or 26, wherein the sample is an optionally diluted crude sample, such as an optionally diluted sample of cell culture supernatant, lysate, serum or plasma.
28. The method according to item 27, wherein the sample is albumin-depleted or non-depleted.
29. The method according to any one of the items 25-28, wherein the at least one protease is trypsin and the cleavage amino acid residue is selected from lysine and arginine.
30. The method according to item 29, wherein the cleavage amino acid residue is selected from lysine and arginine.
31. The method according to any one of the items 25-30, wherein the mass of at least one N atom of the cleavage amino acid residue is 15 Da in the standard peptide and 14 Da in the subsequence of the target protein.
32. The method according to any one of the items 25-31, wherein the cleavage amino acid is selected from lysine and arginine and the only isotope label(s) of the standard peptide is/are one or more, such as one or two, ¹⁵N in the cleavage amino acid.
33. The method according to any one of the items 25-32, wherein each standard peptide consists of 6-35 amino acid residues.
34. The method according to any one of the items 25-33, wherein the mass spectrometry of step c) is tandem mass spectrometry (MS/MS), such as parallel reaction monitoring (PRM), involving filtering of precursor ions using an isolation window of 1-3 Th to obtain a spectrum of fragment ions.

### Brief description of the figures

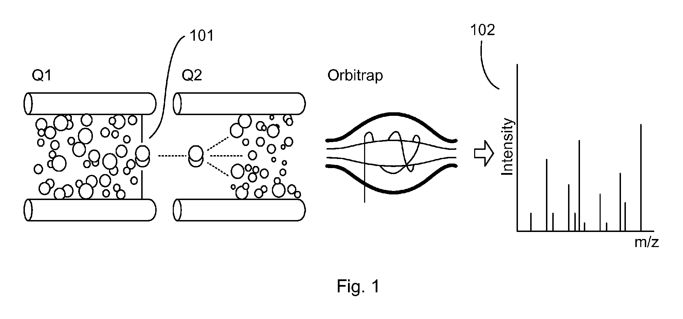

Figure 1 is a simplified scheme of Parallel Reaction Monitoring (PRM) with co-isolation. In the first quadrupole (Q1), precursor ions are selected/filtered using an isolation window (101). In the second quadrupole (Q2), the selected precursor ions are fragmented. In the subsequent analyzer (Orbitrap), fragments from Q2 are detected and a mass spectrum (102) is obtained.

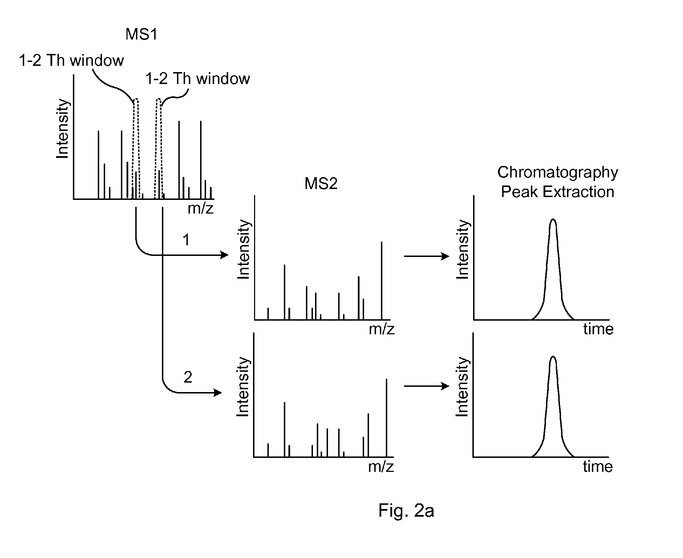

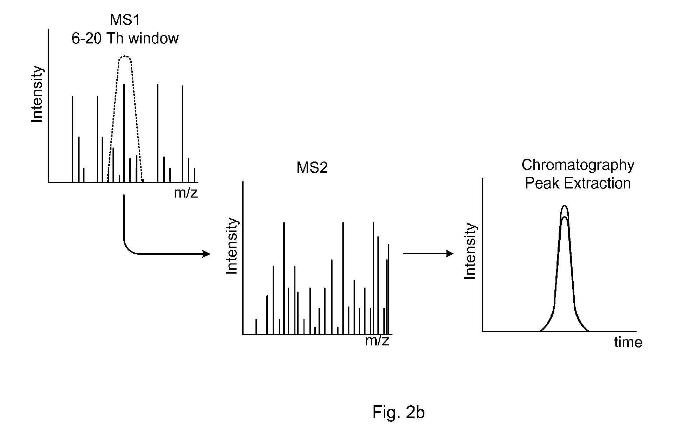

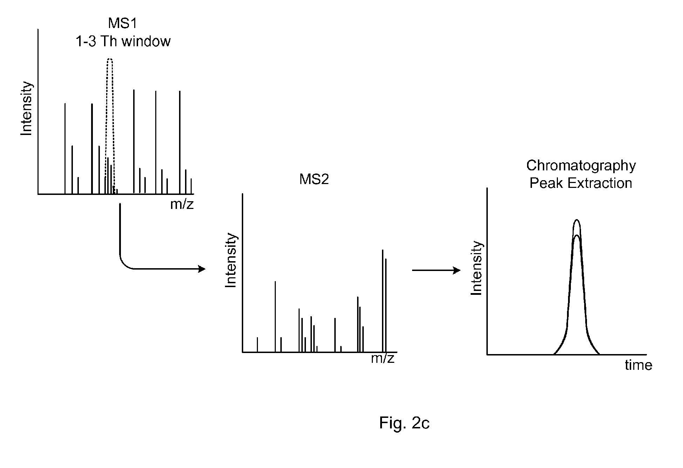

Figure 2 illustrates precursor ion isolation strategies for PRM experiments. In figure 2A, which represents a prior art method, precursor ions are isolated in separate isolation windows such that narrow isolation windows can be used to prevent interfering precursor fragments from reaching the detector. Intensities are scaled in relation to the fill-time used for isolation and ratios between endogenous and standard precursor can be calculated. In figure 2B, which represents another prior art method, a larger window is used to isolate two isotopic precursor ions simultaneously. In figure 2C, the isotopic labeling according to the present disclosure is used and a narrow window of only 1-3 Th is used for precursor co-isolation. Thus, the noise from interfering precursors is decreased and the standard and endogenous precursors can be measured simultaneously.

### Detailed description

As a first aspect of the present disclosure, there is thus provided a method of determining the absolute amount of at least one target protein in a sample. The method comprises the step:
(a) adding an absolute amount of a mass-altered standard protein for each target protein to said sample.

A "target protein" of the present disclosure normally consists of at least 35 amino acid residues. As polypeptides of less than 50 amino acids residues often are referred to a "peptides" rather than "proteins", it is thus understood that the "target protein" of the present disclosure can be a "target peptide" of at least 35 amino acid residues. For example, the target protein may be insulin consisting of 44 amino acid residues.

The amount(s) of the mass-altered standard protein(s) is subsequently used to quantify the target protein(s) (see step (d) discussed below). Each standard protein comprises a target sequence that is identical to a subsequence of its target protein. Further, the target sequence comprises at least one cleavage amino acid residue at which the at least one protease (see step (b) discussed below) cleaves. It follows that the subsequence of the target protein comprises the same at least one cleavage amino acid residue. The mass of each cleavage amino acid residue in the target sequence of the standard protein is altered by 1-5 Da, such as 1-4 Da or 1-2 Da, with respect to the mass of the corresponding cleavage amino acid residue(s) in the subsequence of the target protein. The target sequence of the standard protein is thus cleaved by the at least one protease in the same way as the subsequence of the target protein. The amino acid residues in the target sequence of the standard protein other than the cleavage amino acid residue(s) are not mass altered with respect to the corresponding amino acid residues in the subsequence of the target protein.

The method further comprises the step:
(b) proteolytically cleaving target protein and standard protein from step (a) using at least one protease to obtain cleaved peptides.

If follows from the discussion above that the only difference between cleaved peptides from the target sequence of the standard protein and cleaved peptides from the subsequence of the target protein is the altered mass of the cleavage amino acid residue(s) in the cleaved peptides from the target sequence of the standard protein.

In a preferred embodiment of step (b), the whole sample obtained from step (a) is subjected to the proteolytic cleaving. In such case, not only the target protein and the standard protein are cleaved, but also other proteins of the sample.

The method further comprises the step:
(c) subjecting cleaved peptides from step (b) to mass spectrometry (MS) to obtain a spectrum. Preferably, chromatography, such as liquid chromatography is carried out before the MS in a conventional manner. As the difference in mass between cleaved peptides from the target sequence of the standard protein and cleaved peptides from the subsequence of the target protein is only 1-5 (or 1-4 or 1-2) Da, fragments of each pair of target protein and standard protein can be co-isolated using an isolation window of 1-3 Th, such as 1-2 Th, in the MS.

An example of a suitable MS method for step c) is the "Simultaneous method" described in Gallien et al. (Targeted Proteomic Quantification on Quadrupole-Orbitrap Mass Spectrometer, Molecular & Cellular Proteomics 11 (2012), pages 1709-1723). For example, reference is made to Fig 2 B) of Gallien *et al..* However, it follows from the above that the "Simultaneous method" of Gallien *et al.* has to be modified by setting the isolation window to 1-3 Th and selecting standard proteins comprising one or more cleavage amino acid residues mass-altered by only 1-5 Da, such as 1-4 or 1-2 Da, to achieve the co-isolation.

In the MS, it is preferred to use a mass analyzer having a resolution of at least 30000, such as at least 80000, such as at least 100000.

Examples of high-resolution mass analyzers are an electric field cyclotrone ("Orbitrap"), a magnetic field cyclotron (FT-ICR) and a time-of-flight (TOF) analyzer, such as a Reflector-TOF-Analyzer.

The mass spectrometry of step c) of the method is preferably tandem mass spectrometry (MS/MS). In MS/MS, the so called precursor ions are filtered using the isolation window of 1-3 Th, which means that the obtained spectrum is a spectrum of fragment ions. An example of a suitable tandem mass spectrometry is parallel reaction monitoring (PRM). Gallien *et al.* discusses PRM using an Orbitrap mass analyzer.

The method further comprises the step:
(d) determining the absolute amount of said target protein(s) in said sample from the spectrum obtained in step (c) and said absolute amount(s) of corresponding standard protein(s) added in step (a). Once provided with the teachings of the present disclosure, the skilled person can carry out step (d) without undue burden.

The method of the present disclosure can be used for multiplexing, wherein more than one target protein is quantified and fragments of each pair of target protein and standard protein are co-isolated using an isolation window of 1-3 Th in the MS. Accordingly, the absolute amounts of at least two, such as at least 5, such as at least 10 target proteins are determined in embodiments of the method of the present disclosure.

In complex samples, high amounts of fragments in the mass spectra may interfere with targeted transitions and interfere with their quantification. The present method, which relies on co-isolation with a significantly smaller isolation window than what is normally used for co-isolation, is therefore particularly beneficial in the analysis of complex samples. Accordingly, the sample is a crude sample in one embodiment of the present disclosure. Examples of crude samples are samples of cell culture supernatant, lysate, serum or plasma. A serum or plasma sample may be depleted of albumin. However, the crude sample may also be non-depleted. The crude sample may be diluted or otherwise modified to facilitate the quantification according to the method of the present disclosure.

In one embodiment, the crude sample is diluted in step (a) when the standard protein is added. For example, step (a) may comprise adding buffer, such as bicarbonate buffer, containing the absolute amount of the standard protein.

The sample may be subjected to reduction of disulfide bridges, e.g. by DDT, prior to step (b). The reduction may be followed by alkylation, e.g. with iodoacetic acid (IAA), to prevent that disulfide bridges are formed again. The alkylation is also performed prior to step (b). Preferably, the reduction and the alkylation are performed after the standard protein(s) has/have been added.

The at least one protease of step (b) is for example trypsin, which cleaves at arginine and lysine. Consequently, the at least one cleavage amino acid residue is preferably lysine and arginine when trypsin is used in step (b). To alter the mass of arginine by 1-4 Da, heavy nitrogen (¹⁵N) can be used for 1, 2, 3 or all 4 of the nitrogen atoms of arginine. When all nitrogen atoms of arginine are ¹⁵N, the mass is altered by 4 Da and when two or one of nitrogen atoms of arginine are ¹⁵N, the mass is altered by 2 or 1 Da. Likewise, heavy nitrogen can be used to alter the mass of lysine. When all nitrogen atoms of lysine are ¹⁵N, the mass is altered by 2 Da. When one nitrogen atom of lysine is ¹⁵N, the mass is altered by 1 Da.

Cleavage amino acid residues mass-altered by only 1 or 2 Da allow for an isolation window of only 2 Th or even lower, such as 1-2 Th.

There are however alternatives to the use of lysine and arginine as the cleavage amino acid residues. If chymotrypsin is used in step (b), the at least one cleavage amino acid residue may be selected from tyrosine (Y), phenylalanine (F), tryptophan (W) and leucine (L). If ¹⁵N is used in tyrosine, leucine or phenylalanine, the amino acid residue is mass-aletered by 1 Da. If ¹⁵N is used in tryptophan, the amino acid residue is mass-aletered by 1 or 2 Da.

If Glu-C is used in step (b), the cleavage amino acid residue may be glutamate (E). If ¹⁵N is used in glutamate, the amino acid residue is mass-aletered by 1 Da.

If Lys-C is used in step (b), the cleavage amino acid residue may be lysine (K) only. As mentioned above, the amino acid residue is mass-aletered by 1 or 2 Da if ¹⁵N is used in lysine.

If Arg-C is used in step (b), the cleavage amino acid residue may be arginine (R) only. As mentioned above, the amino acid residue is mass-aletered by 1, 2, 3 or 4 Da if ¹⁵N is used in arginine.

If Asp-N is used in step (b), the cleavage amino acid residue may be aspartate (D). If ¹⁵N is used in aspartate, the amino acid residue is mass-aletered by 1 Da.

In conclusion, the at least one cleavage amino acid may for example be selected from tyrosine, phenylalanine, tryptophan, leucine, lysine, arginine, glutamate and aspartate.

In one embodiment, the at least one cleavage amino acid is selected from lysine, arginine, glutamate and aspartate or only from lysine and arginine.

Each target sequence may for example consist of 15-205 amino acid residues, such as 20-150 amino acid residues, such as 25-150 amino acid residues. When the target sequence is longer, it generally contains more cleavage amino acid residues and thus generates a higher number of cleaved peptides in step (b), which in turn enables a more accurate quantification. For example, each target sequence may comprise at least 2 cleavage amino acid residues, such as at least 3, 4, 5 or 6 cleavage amino acid residues.

The standard protein can be a full-length protein. In such an embodiment, not only a target sequence of the standard peptide is identical to a subsequence of the target protein, the full sequence of the standard peptide is identical to the full sequence of the target protein. The standard protein can also have more or less the same length as the target protein, but include one or several amino acids substitutions, additions or deletions as long as the other requirements of the first aspect are fulfilled.

The standard protein can also be a fusion protein and thus comprise at least one sequence in addition to the target sequence. Accordingly, the standard protein can be longer than the target sequence. For example the standard protein may comprise 35-455 amino acid residues, such as 40-300 amino acid residues, such as 100-200 amino acid residues. As polypeptides of less than 50 amino acids residues often are referred to a "peptides" rather than "proteins", it is thus understood that the "standard protein" of the present disclosure can be a "standard peptide" of at least 35 amino acid residues.

When the standard protein is a fusion protein, it may comprise a solubility tag or a fragment thereof, wherein the solubility tag is selected from Maltose-binding protein (MBP), Glutathione-S-transferase (GST), Thioredoxin (Trx), N-Utilization substance (NusA), Small ubiquitin-modifier (SUMO), a Solubility-enhancing tag (SET), a Disulfide forming protein C (DsbC), Seventeen kilodalton protein (Skp), Phage T7 protein kinase (T7PK), Protein G B1 domain (GB1), Protein A IgG ZZ repeat domain (ZZ) and Albumin Binding Protein (ABP).

The solubility tag or the fragment thereof preferably consists of at least 15 amino acid residues, such as at least 25 amino acid residues. In one embodiment, the maximum length of the solubility tag or the fragment thereof is 200 amino acid residues.

When the standard protein is a fusion protein, it may also comprise a purification tag selected from a His tag, a FLAG tag, a SBP tag, a myc tag and a OneStrep tag.

As a second aspect of the present disclosure, there is provided an isotopically labelled fusion protein for the quantification of a target protein by mass spectrometry. The isotopically labelled fusion protein of the second aspect is designed to be used in the method of the first aspect. A "target protein" of the present disclosure may be a human protein. Accordingly, the method of the first aspect and the isotopically labelled fusion protein of the second aspect can be used clinically, e.g. for diagnostic purposes.

The fusion polypeptide of the second aspect consists of 30-455 amino acid residues and comprises:
(i) a target sequence, which is identical to the target protein or a subsequence thereof, and
(ii) a second sequence, which is not identical to the target protein or a subsequence thereof.

This means that the target sequence and the second sequence cannot be two subsequences of the same target protein.

The target sequence of the second aspect consists of 15-205 amino acid residues and comprises at least one amino acid residue mass-altered by 1-2 Da. This is achieved by selecting the at least one mass-altered amino acid residue from the group consisting of lysine and arginine in which one or two of the nitrogen atoms is/are ¹⁵N.

The amino acid residues in the target sequence other than lysine and arginine amino acid residues are not mass-altered. When the fusion protein of the second aspect is used as a standard protein in the first aspect, an isolation window 2 Th or lower can thus be used in the co-isolation.

The second sequence of the fusion protein of the second aspect consists of 15-250 amino acid residues, such as 20-250 amino acid residues, such as 25-250 amino acid residues.

The embodiments of the first aspect apply to the second aspect *mutatis mutandis.* However, some embodiments of the second aspect are anyway discussed below.

The fusion protein of the second aspect preferably comprises at least 2, such as at least 3, 4, 5 or 6 amino acid residues mass-altered by 1-2 Da. It is understood that such mass-altered amino acid residues are selected from lysine and arginine in which one or two of the nitrogen atoms is/are ¹⁵N.

In the prior art, arginine that is isotope-labeled with ¹⁵N normally has four ¹⁵N isotopes. In contrast, the arginine residues of the target sequence of the second aspect only have one or two ¹⁵N isotopes.

Preferably, all of the lysine and arginine residues of the target sequence are mass altered by 1-2 Da by incorporation of 1 or 2 ¹⁵N.

In one embodiment, the fusion protein of the second aspect consists of at least 40 amino acid residues. For example, it may consist of 40-300 amino acid residues, such as 100-250 amino acid residues. The target sequence may for example consist of 20-200 amino acid residues, such as 30-170 amino acid residues, such as 50-150 amino acid residues.

The second sequence of the second aspect is preferably a functional sequence. For example, the second sequence may be a solubility tag or a fragment thereof. The solubility tag can for example be selected from Maltose-binding protein (MBP), Glutathione-S-transferase (GST), Thioredoxin (Trx), N-Utilization substance (NusA), Small ubiquitin-modifier (SUMO), a Solubility-enhancing tag (SET), a Disulfide forming protein C (DsbC), Seventeen kilodalton protein (Skp), Phage T7 protein kinase (T7PK), Protein G B1 domain (GB1), Protein A IgG ZZ repeat domain (ZZ) and Albumin Binding Protein (ABP).

As understood from the above, a solubility fragment cannot be shorter than 15 amino acid residues if it constitutes the second sequence.

The fusion protein of the second aspect can further comprise a purification tag, such as a tag selected from a His tag, a FLAG tag, a SBP tag, a myc tag and a OneStrep tag.

As a third aspect of the present disclosure, there is provided a method of determining the absolute amount of at least one target protein in a sample, said method comprising the following steps:
(a) proteolytically cleaving target protein from or in the sample using at least one protease to obtain cleaved peptides, wherein an absolute amount of a mass-altered standard peptide for each target protein is added before or after the proteolytic cleaving such that a mixture of cleaved peptides and standard peptide(s) is obtained after the proteolytic cleaving;
(b) subjecting the mixture, optionally after chromatography, to mass spectrometry (MS) to obtain a spectrum, which MS comprises filtering using an isolation window of 1-3 Th for co-isolation of ions from each pair of target protein and standard peptide; and
(c) determining the absolute amount of said target protein(s) in said sample from the spectrum obtained in step (b) and said absolute amount(s) of corresponding standard peptide(s) added in step (a), wherein
   (i) the amino acid sequence of each standard peptide is identical to a subsequence of its target protein and comprises at one of the terminal ends a cleavage amino acid residue at which the at least one protease cleaves,
   (ii) the mass of the cleavage amino acid residue in the standard peptide is altered by 1-5 Da, such as 1-4 Da or 1-2 Da, with respect to the mass of the corresponding cleavage amino acid residue(s) in the subsequence of the target protein and
   (iii) the amino acid residues in the standard peptide other than the cleavage amino acid residue are not mass altered with respect to the corresponding amino acid residues in the subsequence of the target protein.

The first aspect and the third aspect are based on the same inventive insights. The main difference between the first aspect and the third aspect is that the standard peptide(s) of the third aspect is/are not cleavable by the at least one protease used in the proteolytic step. Instead, the standard peptide(s) of the third aspect already has/have the sequence of a cleaved product, while the standard protein(s) of the first aspect is/are designed to be cleaved. As a consequence, the standard peptide(s) of the third aspect can be added before or after (preferably after) the proteolytic step, while the standard protein(s) of the first aspect has/have to be added before the proteolytic step.

It follows from the above that if the at least one protease cleaves on the carboxyl side of the cleavage amino acid residue, the cleavage amino acid residue constitutes the C terminal end of the standard peptide and if the at least one protease cleaves on the amino side of the cleavage amino acid residue, the cleavage amino acid residue constitutes the N terminal end of the standard peptide.

Trypsin cleaves on the carboxyl side of arginine (R) and lysine (K) residues. Accordingly, arginine or lysine constitutes the C terminal end of each standard peptide when the at least one protease is trypsin.

Chymotrypsin cleaves on the carboxyl side of tyrosine (Y), phenylalanine (F), tryptophan (W) and leucine (L) residues. Accordingly, tyrosine, phenylalanine, tryptophan or leucine constitutes the C terminal end of each standard peptide when the at least one protease is chymotrypsin.

Lys-C cleaves on the carboxyl side of lysine (K) residues. Accordingly, lysine constitutes the C terminal end of each standard peptide when the at least one protease is Lys-C.

Glu-C cleaves on the carboxyl side of glutamate (E) residues. Accordingly, glutamate constitutes the C terminal end of each standard peptide when the at least one protease is Glu-C.

Arg-C cleaves on the carboxyl side of arginine (R) residues. Accordingly, arginine constitutes the C terminal end of each standard peptide when the at least one protease is Arg-C.

Asp-N cleaves on the amino side of aspartate (D) residues. Accordingly, aspartate constitutes the N terminal end of each standard peptide when the at least one protease is Asp-N.

In one embodiment of the third aspect, each standard peptide consists of 4-35 amino acid residues, such as 6-35 amino acids residues, such as 6-25 amino acid residues, such as 6-15 amino acid residues. A lower limit of 6 amino acids may be preferred to reduce the risk of including unrelated peptides in the quantification.

It may be beneficial to avoid chemically reactive residues or chemically unstable sequences in the standard peptide. Accordingly, each standard peptide may lack tryptophan, methionine and/or cysteine. Further, each standard peptide may lack an Asp-Gly sequence, an N-terminal Gln and/or an N-term Asn.

Otherwise, the embodiments of the first aspect apply to the third aspect *mutatis mutandis.*

### Example

### Stable-isotope labeled standards

Stable isotope labeled (SIL) fusion protein standards containing commercially available heavy isotope labeled Arginine and Lysine (1-2 ¹⁵N isotope-labeled) are prepared, pre-quantified against a quantification tag, and used as internal standards for MS-based absolute quantification. Here, the introduction of one ¹⁵N to Arg or Lys alters the mass of standard peptides after trypsin digestion by only 0.997 Da when compared to the mono-isotopic [M+0] peak of the endogenous unlabeled peptides originating from the biological sample, while introduction of two ¹⁵N gives a mass shift of 1.994 Da.

### Sample preparation

Samples of 1 µl non-depleted human plasma are diluted in 9 µl 20 mM ammonium bicarbonate buffer containing known absolute amounts of SIL fusion protein standards that have been added in a ratio of approximately 1:1 to the expected plasma concentration of the corresponding endogenous proteins. The samples are then incubated 15 minutes at 95 °C. Reduction is performed by addition of DTT to a final concentration of 10 mM and incubated for 30 minutes at 56 °C followed by alkylation with 55 mM IAA 30 minutes at room temperature. Porcine trypsin (MS-grade, Sigma Aldrich) is added in a 1:50 enzyme to substrate ratio and digestion is performed overnight at 37 °C. The enzyme activity is quenched by addition of formic acid to a final concentration of 0.1%. Peptides are desalted and concentrated using C18 stage tips and eluted using 60 % Acetonitrile (ACN) 0.1 % formic acid (FA). Peptides and elution buffer are dried down in a vacuum concentrator followed by re-suspension by addition of 3% ACN, 0.1 % FA prior to LC-MS analysis using Parallel Reaction Monitoring.

### Parallel Reaction Monitoring (PRM)

PRM experiments are performed on a Q Exactive HF (Thermo Scientific) high-resolution mass spectrometer coupled to a Dionex Nano Ultimate 3000 liquid chromatography system with an Easy Nano-nLC 1000 ion source.

Samples are separated by reverse phase on a C18-column (Pepmap 3µm, 15cm x 75µm, Thermo Scientific) using a 15-minute linear gradient with increasing part Buffer B (ACN, 0.1 % Formic acid) from 3% to a final concentration of 35 %.

A scheduled PRM assay is set up containing the liquid chromatography retention time precursor ion mass-to-charge (m/z) of the target peptides. The retention time windows are chosen to start 2 minutes before and end 2 minutes after the expected elution of each target peptide in order to account for potential drift in the chromatography. In order to co-isolate both precursor ions from heavy SIL standards and light endogenous, the mean value for these ions is used to center the isolation window. The isolation window is set to 1.5 or 2 Th for precursor ions containing 1 or 2 ¹⁵N atoms, respectively. This isolation window is big enough to allow for co-isolation of both labeled and unlabeled m/z++ precursor ions with 0.5 Th margins on each side of the precursors. However, the window is also small enough to prevent too many non-relevant precursor ions to be co-isolated, as would be the case with larger isolation windows, such as windows of 5 Th or more.

The scheduled PRM is performed at a high resolution (120,000 @ 200 m/z) with a maximum fill time of 500 ms. The normalized collision energy is set to 30 for all precursors.

### Data analysis

Raw data files are analyzed using the Skyline software and target transition ions are extracted using an in-house developed spectral library. Ratios of integrated peak areas of y-ion fragments from heavy and light peptides are used to calculate the concentration of protein in the analyzed plasma samples from the known absolute amounts of SIL fusion protein standards added during sample preparation.

## Claims

1. A method of determining the absolute amount of at least one target protein in a sample, said method comprising the following steps:
(a) adding an absolute amount of a mass-altered standard protein for each target protein to said sample;
(b) proteolytically cleaving target protein and standard protein from step (a) using at least one protease to obtain cleaved peptides;
(c) subjecting cleaved peptides from step (b), optionally after chromatography, to mass spectrometry (MS) to obtain a spectrum, which MS comprises filtering using an isolation window of 1-3 Th to co-isolate ions from each pair of target protein and standard protein; and
(d) determining the absolute amount of said target protein(s) in said sample from the spectrum obtained in step (c) and said absolute amount(s) of corresponding standard protein(s) added in step (a), wherein
(i) each standard protein comprises a target sequence that is identical to a subsequence of its target protein,
(ii) the target sequence comprises at least one cleavage amino acid residue at which the at least one protease cleaves,
(iii) the mass of each cleavage amino acid residue in the target sequence of the standard protein is altered by 1-5 Da, such as 1-4 Da or 1-2 Da, with respect to the mass of the corresponding cleavage amino acid residue(s) in the subsequence of the target protein and
(iv) the amino acid residues in the target sequence of the standard protein other than the cleavage amino acid residue(s) are not mass altered with respect to the corresponding amino acid residues in the subsequence of the target protein.

2. A method of determining the absolute amount of at least one target protein in a sample, said method comprising the following steps:
(a) proteolytically cleaving target protein from or in the sample using at least one protease to obtain cleaved peptides, wherein an absolute amount of a mass-altered standard peptide for each target protein is added before or after the proteolytic cleaving such that a mixture of cleaved peptides and standard peptide(s) is obtained after the proteolytic cleaving;
(b) subjecting the mixture, optionally after chromatography, to mass spectrometry (MS) to obtain a spectrum, which MS comprises filtering using an isolation window of 1-3 Th for co-isolation of ions from each pair of target protein and standard peptide; and
(c) determining the absolute amount of said target protein(s) in said sample from the spectrum obtained in step (b) and said absolute amount(s) of corresponding standard peptide(s), wherein
(i) the amino acid sequence of each standard peptide is identical to a subsequence of its target protein and comprises at one of the terminal ends a cleavage amino acid residue at which the at least one protease cleaves,
(ii) the mass of the cleavage amino acid residue in the standard peptide is altered by 1-5 Da, such as 1-4 Da or 1-2 Da, with respect to the mass of the corresponding cleavage amino acid residue(s) in the subsequence of the target protein and
(iii) the amino acid residues in the standard peptide other than the cleavage amino acid residue are not mass altered with respect to the corresponding amino acid residues in the subsequence of the target protein.

3. The method according to claim 1 or 2, wherein the absolute amounts of at least two, such as at least 5, such as at least 10 target proteins are determined.

4. The method according to any one of the preceding claims, wherein the sample is an optionally diluted crude sample, such as an optionally diluted sample of cell culture supernatant, lysate, serum or plasma.

5. The method according to any one of the preceding claims, wherein the at least one protease is trypsin and the at least one cleavage amino acid residue is selected from lysine and arginine.

6. The method according to claim 1, wherein the mass of at least one N atom of the at least one cleavage amino acid residue is 15 Da in the target sequence of the standard protein and 14 Da in the subsequence of the target protein.

7. The method according to claim 1 or 6, wherein the only isotope labels of the target sequence of each standard protein are one or more ¹⁵N in each lysine and arginine.

8. The method according to any one of claims 1, 6 and 7, wherein each target sequence consists of 15-205 amino acid residues, such as 20-120 amino acid residues, such as 30-170 amino acid residues, such as 50-150 amino acid residues.

9. The method according to any one of claims 1, 6, 7 and 8, wherein each target sequence comprises at least 2 cleavage amino acid residues, such as at least 3, 4, 5 or 6 cleavage amino acid residues.

10. The method according to any one of claims 1, 6, 7, 8 and 9, wherein the standard protein is a fusion protein.

11. The method according to claim 10, wherein the fusion protein comprises a solubility tag or a fragment thereof, wherein the solubility tag is selected from Maltose-binding protein (MBP), Glutathione-S-transferase (GST), Thioredoxin (Trx), N-Utilization substance (NusA), Small ubiquitin-modifier (SUMO), a Solubility-enhancing tag (SET), a Disulfide forming protein C (DsbC), Seventeen kilodalton protein (Skp), Phage T7 protein kinase (T7PK), Protein G B1 domain (GB1), Protein A IgG ZZ repeat domain (ZZ) and Albumin Binding Protein (ABP).

12. The method according to any one of the preceding claims, wherein the mass spectrometry of step c) is tandem mass spectrometry (MS/MS), such as parallel reaction monitoring (PRM), involving filtering of precursor ions using an isolation window of 1-3 Th to obtain a spectrum of fragment ions.

13. An isotopically labelled fusion protein for the quantification of a target protein by mass spectrometry, wherein:
said fusion polypeptide consists of 30-455 amino acid residues and comprises a target sequence, which is identical to the target protein or a subsequence thereof, and a second sequence, which is not identical to the target protein or a subsequence thereof;
said target sequence consists of 15-205 amino acid residues and comprises at least one amino acid residue mass-altered by 1-2 Da, which mass-altered amino acid residue(s) is/are selected from lysine and arginine in which one or two of the nitrogen atoms is/are ¹⁵N, wherein the amino acid residues in the target sequence other than lysine and arginine amino acid residues are not mass-altered; and
said second sequence consists of 15-250 amino acid residues, such as 20-250 amino acid residues, such as 25-250 amino acid residues.

14. The isotopically labelled fusion protein of claim 13, wherein said target sequence comprises at least 2, such as at least 3, 4, 5 or 6 amino acid residues mass-altered by 1-2 Da, which mass-altered amino acid residues are selected from lysine and arginine in which one or two of the nitrogen atoms is/are ¹⁵N.

15. The isotopically labelled fusion protein of claim 13 or 14, wherein said target sequence comprises at least one arginine residue in which one or two of the nitrogen atoms is/are ¹⁵N.
